# EUROPÄISCHE PATENTANMELDUNG

(11) **EP 3 050 538 A1**
(43) Veröffentlichungstag der Anmeldung: **03.08.2016**
(21) Anmeldenummer: 15152592.0
(22) Anmeldetag: 27.01.2015
(51) Int. Cl.: A61F 2/34

(54) **Pfanne einer Hüftgelenkprothese**

(71) Anmelder: AQ Implants GmbH, 22926 Ahrensburg (DE)
(72) Erfinder: Drenckhan, Sylke, 23556 Lübeck (DE)
(74) Vertreter: Grünecker Patent- und Rechtsanwälte PartG mbB

(57) **Zusammenfassung**

Die vorliegende Erfindung betrifft eine Pfanne (8) einer Hüftgelenkprothese mit einer inneren Aufnahme für die drehbare Lagerung eines Oberschenkels und mit einer das Einwachsen von Knochenzellen begünstigenden Oberflächengestaltung (4) und will eine Pfanne (8) angeben, deren Oberflächengestaltung (4) in verbesserter Weise das Einwachsen von Knochenzellen begünstigt. Dazu umfasst die Oberflächengestaltung (4) Stege (14), die die Mantelfläche von Pyramiden begrenzen, deren Grundfläche im Wesentlichen parallel zu der durchgehenden Oberfläche (6) verläuft, von der die Stege (14) abragen.

## Beschreibung

Die vorliegende Erfindung betrifft im Allgemeinen prothetische Implantate und bezieht sich insbesondere auf das Implantat von Hüftgelenkspfannenanordnungen, die ein prothetisches Lagerelement in der Hüftgelenkspfanne zur Aufnahme eines Oberschenkelkopfes eines Hüftgelenkes befestigen.

Die vorliegende Erfindung betrifft insbesondere eine Pfanne eines solchen prothetischen Lagerelements mit einer inneren Aufnahme für die drehbare Lagerung eines Oberschenkels und mit einer das Einwachsen von Knochenzellen begünstigenden Oberflächengestaltung, die über einer äußeren durchgehenden Oberfläche der Pfanne vorgesehen ist. Eine solche Pfanne mit einer das Einwachsen von Knochenzellen begünstigenden Oberflächengestaltung ist beispielsweise aus der WO 2008/146141 A2 bekannt.

Die vorliegende Erfindung will eine solche Pfanne angeben, deren Oberflächengestaltung in verbesserter Weise das Einwachsen von Knochenzellen begünstigt.

Zur Lösung des obigen Problems der vorliegenden Erfindung wird eine Pfanne mit den Merkmalen von Anspruch 1 vorgeschlagen. Diese zeichnet sich dadurch aus, dass die Oberflächengestaltung Stege umfasst, die die Mantelfläche von Pyramiden begrenzen, deren Grundfläche im Wesentlichen parallel zu der durchgehenden Oberfläche verläuft, von der die Stege abragen.

Die Oberflächengestaltung hat dementsprechend Zellen, die innerhalb der Pyramide vorgesehen sind. Dabei kann es sich auch um eine Doppelpyramide handeln, die auf der durchgehenden Oberfläche aufgebracht ist. Eine besondere Form der Doppelpyramide ist dabei der Oktaeder, bestehend aus drei senkrecht zueinander stehenden Quadraten.

Die Stege einer Pyramide bilden eine Pyramidenspitze aus, die vorzugsweise mit der durchgehenden Oberfläche verbunden ist. In einem solchen Fall liegt die Grundfläche einer solcherart ausgebildeten Pyramide mit Abstand zu der durchgehenden Oberfläche, erstreckt sich hierzu aber im Wesentlichen parallel. Es versteht sich von selbst, dass die durchgehende Oberfläche einer Pfanne üblicherweise sphärisch ist. Demgegenüber sind die Stege vorzugsweise geradlinig verlaufend, d. h. spannen in einer Ebene liegende Rechtecke, vorzugsweise Quadrate, auf.

Die durch die Pyramiden gebildeten Zellen begünstigen einerseits das Einwachsen von Knochenzellen. Darüber hinaus ergeben sich durch die gradlinigen und üblicherweise sich zu viert in einem Punkt auf Höhe der durchgehenden Oberfläche schneidenden Stege Hinterschnitte welche die darin eingewachsenen Knochenzellen formschlüssig gegenüber der Oberflächengestaltung der Pfanne verriegeln und damit sicher die Knochenzellen mit der Pfanne verbinden. Sofern die Pyramide als Doppelpyramide ausgebildet ist, ergeben sich solche Hinterschnitte auf zwei übereinander liegenden Ebenen, nämlich zwischen der Grundfläche und der durchgehenden Oberfläche unter den von einer unteren Spitze der unteren Pyramide nach außen konvergierenden Stegen einerseits sowie zwischen der Grundfläche und der mit maximalem Abstand von der durchgehenden Oberfläche vorgesehenen Pyramidenspitze andererseits.

Eine verbesserte Verankerung der Knochenzellen an der Oberflächengestaltung und eine solidere Zellenstruktur, die das Einwachsen von Knochenzellen ermöglicht und durch die Stege der Oberflächengestaltung gebildet wird, ist dadurch geschaffen, dass die Spitze von zwei senkrecht zueinander stehenden Rechtecken der Doppelpyramide, im Spezialfall eines Oktaeders von senkrecht zueinander stehenden Quadraten gebildet wird. Ein drittes zu diesen beiden Rechtecken bzw. Quadraten senkrecht stehendes Rechteck erstreckt sich im Wesentlichen parallel und mit Abstand zu der durchgehenden Oberfläche. Das dritte Rechtseck bzw. Quadrat ist vorzugsweise allein mit seinen Eckpunkten definiert, die durch die Eckpunkte der ersten und zweiten Rechtecke gebildet sind. Auf Höhe des dritten Rechtecks, d.h. auf mittlerer Höhe der Doppelpyramide, welches die Grundfläche der beiden Pyramiden ausbildet, befinden sich damit keine sich in dieser Ebene erstreckende Stege. Das dritte Rechteck und damit die Grundfläche ist damit vorzugsweise lediglich durch die gedachte Hüllfläche umschrieben, die durch die Eckpunkte desselben miteinander verbindende Geraden definiert ist.

Benachbarte Doppelpyramiden sind vorzugsweise auf Höhe des dritten Rechtecks miteinander verbunden sind. In einer vereinfachten Anschauung kann man sich vorstellen, dass jeweils die dritten Rechtecke in Umfangsrichtung bzw. Höhenrichtung und auf halber Höhe der Doppelpyramide üblicherweise benachbarte dritte Rechtecke bzw. Quadrate benachbarter Doppelpyramiden haben. Eine von der durchgehenden Oberfläche abragende Doppelpyramide hat jeweils in Umfangsrichtung zumindest zwei benachbarte Doppelpyramiden und in Höhenrichtung eine entsprechende Anzahl von benachbarten Doppelpyramiden. Als Umfangsrichtung im Sinne der vorliegenden Erfindung soll eine Erstreckungsrichtung parallel zu derjenigen Ebene verstanden werden, zu welcher sich die innere Aufnahme für die drehbare Lagerung des Oberschenkels öffnet. Der sich senkrecht dazu erstreckende Weg von dieser Aufnahme bis hin zu dem Boden der Pfanne auf deren Außenumfangsfläche soll als Höhenrichtung verstanden werden.

Gemäß der oben diskutierten Weiterbildung sind die dritten Rechtecke bzw. Quadrate, d. h. die Grundflächen der Doppelpyramiden miteinander verbunden und zwar vorzugsweise in Umfangsrichtung der Pfanne. So sind in Umfangsrichtung der Pfanne nebeneinander angeordnete Pyramiden über das dritte Rechteck bzw. Quadrat miteinander verbunden. Die Verbindung erfolgt dabei vorzugsweise allein über die Eckpunkte der dritten Rechtecke. Zwei benachbarte Grundflächen haben somit zwei gemeinsame Eckpunkte.

Durch diese Ausgestaltung ergeben sich gedachte, in Umfangsrichtung umlaufende Sprossenstrukturen, die in Höhenrichtung versetzt sind, wobei die Eckpunkte der dritten Rechtecke bzw. Quadrate die Knotenpunkte und damit Gitternetzpunkte dieser umlaufenden, gedachten Sprossenstruktur bilden. Dabei liegen in Umfangsrichtung benachbarte Gitternetzpunkte einer einzigen, in Umfangsrichtung umlaufenden Sprossenstruktur auf einer streng in Umfangsrichtung umlaufenden Linie und die beiden in Höhenrichtung benachbarten Gitternetzpunkte dieser umlaufenden Sprossenstruktur streng in Höhenrichtung nebeneinander. Die Gitternetzpunkte einer einzigen Sprossenstruktur laufen mit radialem Abstand um die durchgehende Oberfläche der Pfanne um. Der radiale Abstand der Gitternetzpunkte von der durchgehenden Oberfläche beträgt vorzugsweise maximal zwischen 1 und 2 mm. Zwischen den Gitternetzpunkten und der durchgehenden Oberfläche befindet sich dabei üblicherweise eine Pyramide, wohingegen eine Pyramide üblicherweise gleicher Abmessung radial außerhalb der Sprossenstruktur sich von dieser erhebt. Der besagte Abstand zwischen der durchgehenden Oberfläche und den Gitternetzpunkte einer einzigen Sprossenstruktur kann in Umfangsrichtung annähernd konstant sein. Ebenso gut kann der radiale Abstand zwischen den Gitternetzpunkten der umlaufenden Sprossenstruktur und der durchgehenden Oberfläche wellenförmig ausgebildet sein, so dass in gewissen Umfangsabschnitten sich die Sprossenstruktur der durchgehenden Oberfläche annähert, in anderen Bereichen aber Abstand zu dieser durchgehenden Oberfläche gewinnt. Die Grundfläche und der radiale Abstand der Gitterpunkte einer einzigen, vorzugsweise aller umlaufender Sprossenstrukturen ist regelmäßig so gewählt, dass die Sprossenstruktur absatzfrei bei konstanter Grundfläche je umlaufender Sprossenstruktur um die durchgehende Fläche umläuft; bei konstantem Radius der Sprossenstruktur ergibt sich dieser damit als ganzzahliges Vielfaches der Erstreckung der Grundfläche, d.h. der zugeordneten Gitternetzpunkte in Umfangsrichtung.

Soweit vorstehend auf einen maximalen Abstand abgestellt wurde bedeutet dies, dass dieser maximale Abstand zwischen den Gitternetzpunkten der Grundfläche von in Umfangsrichtung umlaufenden Doppelpyramiden und der durchgehenden Oberfläche üblicherweise nicht überschritten wird. Vorzugsweise nimmt dieser Abstand mit zunehmender Annäherung der jeweiligen umlaufenden Sprossenstruktur an den Boden der Pfanne ab. So kann beispielsweise der Abstand in Umfangsrichtung für jeden einzelnen Ring der Sprossenstruktur nach obiger Maßgabe nahe der Eingangsebene zu der inneren Aufnahme zunächst identisch sein. Mit zunehmendem Abstand von der Eingangsebene zu der inneren Aufnahme nimmt dieser Abstand indes ab. Dabei kann der Abstand kontinuierlich in Richtung auf den Rand der Pfanne abnehmen. Bevorzugt ist indes, den Abstand bis zu einer Höhe von zwischen 50 bis 70 Prozent der Gesamthöhe unverändert zu belassen und erst in dem verbleibenden Höhenanteil der Pfanne den Abstand zu reduzieren. Der Abstand kann sich im Bereich des Bodens Null annähern. Mit anderen Worten können im Bereich des Bodens sehr kleine, auf einer Kreisbahn umlaufende Pyramiden mit Gitternetzpunkten, die einen Abstand von wenigen Zehntel mm von der durchgehenden Oberfläche haben, oder keine Pyramiden vorgesehen sein. Mit Blick auf diese Höhenabnahme der das Einwachsen von Knochenzellen begünstigenden Oberflächenstruktur in Richtung auf den Boden der Pfanne kann sich auch die Gestaltung der einzelnen Zellen ändern. Während im Bereich der Eingangsebene Doppelpyramiden vorgesehen sein können, mag im Bereich des Pfannenbodens lediglich eine einfache Pyramide vorgesehen sein, die indes ein durch Stege umfänglich geschlossenes Rechteck als Grundfläche aufweisen kann. Bei sich verändernder Zellgröße wird vorzugsweise bei sämtlichen Sprossenstrukturen das Verhältnis von Abstand zu Steglänge in Umfangsrichtung und/oder Höhenrichtung der rechteckigen Grundflächen im Wesentlichen konstant gehalten. Dabei sind die Steglängen bei quadratischer Grundfläche sowohl in Umfangsrichtung wie auch in Höhenrichtung identisch. Wird der Abstand der jeweiligen Grundfläche von der durchgehenden Oberfläche der Pfanne dementsprechend geringer, ergeben sich auch kleinere Zellen bedingt durch eine verringerte Länge der jeweiligen Sprossen in Höhen- bzw. Umfangsrichtung.

Vorzugsweise umfasst die das Einwachsen von Knochenzellen begünstigende Oberfläche ausschließlich Stege, die die Mantelfläche von Pyramiden begrenzen. Alle Zellen sind damit durch Pyramiden oder Doppelpyramiden definiert.

Bedingt durch das bevorzugte Fertigungsverfahren zur Herstellung der das Einwachsen von Knochenzellen begünstigenden Oberflächengestaltung einheitlich mit der Pfanne durch generative/additive Fertigungsverfahren ist es indes zu bevorzugen, einen überwiegenden Teil der einzelnen Pyramiden bzw. Doppelpyramiden auf der Oberfläche der Pfanne identisch auszubilden. Da in Höhenrichtung der Pfanne der tatsächlich mit der Oberflächengestaltung zu versehende Radius abnimmt, wird gemäß einer bevorzugten Weiterbildung der vorliegenden Erfindung vorgeschlagen, in Höhenrichtung benachbarte Reihen von Doppelpyramiden nicht miteinander zu verbinden.

Nicht nur die auf der durchgehenden Oberfläche in Form von Zellen vorgesehene Oberflächengestaltung begünstigt das Einwachsen von Knochenzellen, sondern auch die Morphologie der durchgehenden Oberfläche als solches. Mit Blick darauf wird vorgeschlagen, die durchgehende Oberfläche mit einer Rauigkeit Rₐ von zwischen 4 und 40 zu versehen. Diese Oberfläche kann durch Strahlen aufgeraut sein, um beispielsweise eine Rauigkeit Rₐ von zwischen 4 und 6 erreichen. Ebenso gut kann die Oberfläche mittels Plasmasprayen modifiziert sein, um eine Oberflächenrauigkeit eher im oberen Bereich des beanspruchten Intervalls zu verwirklichen.

Weitere Einzelheiten und Vorteile der vorliegenden Erfindung ergeben sich aus der nachfolgenden Beschreibung eines Ausführungsbeispiels in Verbindung mit der Zeichnung. In dieser zeigen:
- Figur 1: eine perspektivische Seitenansicht einer Doppelpyramide als Beispiel für eine Zelle dieser das Einwachsen von Zellen verbessernder Oberflächenstruktur;
- Figur 2: eine perspektivische Seitenansicht eines Beispiels einer Pfanne der vorliegenden Erfindung; und
- Figur 3: eine Seitenansicht des Beispiels nach Figur 3.

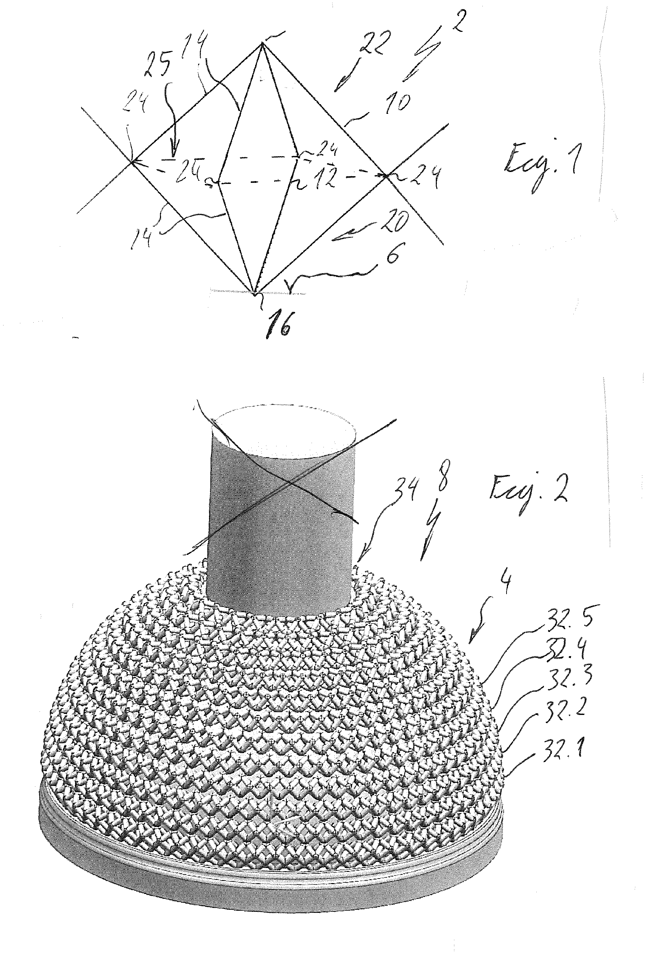

Figur 1 zeigt eine schematisch-perspektivische Seitenansicht einer Struktur 2, welche die kleinste Zelle einer in Figur 2 insgesamt mit Bezugszeichen 4 gekennzeichneten Oberflächengestaltung darstellt, die auf einer durchgehenden Oberfläche 6 einer in Figur 2 mit Bezugszeichen 8 gekennzeichneten Pfanne ausgebildet ist. Die Struktur 2 ist eine Doppelpyramidenstruktur mit einem ersten Quadrat 10 und einen zweiten Quadrat 12, die jeweils eine sich rechtwinklig zu der durchgehenden Oberfläche 6 erstreckende Fläche aufspannen und rechtwinklig zueinander ausgerichtet sind. Die Quadrate 10, 12 werden jeweils durch geradlinig verlaufende identische Stege 14 aufgespannt, die im Schnittpunkt der beiden Quadrate 10, 12 eine untere, mit der durchgehenden Oberfläche 6 verbundene Spitze 16 und an ihrem gegenüberliegenden Ende eine obere Spitze 18 ausbilden. Die gezeigten Stege 14 begrenzen die Mantelfläche einer unteren Pyramide 20 bzw. und einer oberen Pyramide 22. Die beiden Pyramiden 20, 22 enden jeweils an den Schnittpunkten der Stege 14 des gleichen Quadrats 10 bzw. 12. Allerdings ist auf dieser mittleren Höhe der gezeigten Doppelpyramide über der durchgehenden Oberfläche kein drittes Quadrat aus Stegen umhüllt, welche die dort befindlichen Eckpunkte der beiden Quadrate 10, 12 miteinander verbinden. Auf dieser Höhe wird die Grundfläche der Doppelpyramide aufgespannt, die indes nicht umfänglich durch Stege begrenzt ist, sondern sich als Hüllfläche durch gedachte Verbindungslinien ergibt, die die mit Bezugszeichen 24 gekennzeichneten Eckpunkte der Doppelpyramide 20, 22 verbinden.

Die Stege 14 haben üblicherweise eine Stegbreite von zwischen 0,6 und 0,8 mm, bevorzugt von etwa 0,7 mm. Die Höhe der Doppelpyramide, d. h. der Abstand von der durchgehenden Oberfläche 6 bis zu der oberen Spitze 18 kann dabei maximal 3 mm sein.

Wie die Figur 2 verdeutlicht, ist diese Oberflächenstruktur 4 im Grunde auf der gesamten durchgehenden Oberfläche 6 ausgebildet. In etwa auf Höhe einer mit Bezugszeichen 26 gekennzeichneten Eingangsebene der Pfanne 8 ist zunächst ein Ring 28 umlaufend vorgesehen, der über eine ebenfalls umlaufende Profilierung 30 in denjenigen Bereich übergeht, in dem die das Einwachsen von Knochenzellen begünstigende Oberflächengestaltung 6 ausgebildet ist. Die Eingangsebene 26 ist dabei diejenige Ebene, zu der sich eine nicht zu erkennende innere Aufnahme für die drehbare Lagerung eines Oberschenkels öffnet.

Diese Oberflächengestaltung 4 umfasst mehrere umlaufende Reihen 32, die in Umfangsrichtung durchgehend ausgebildet sind und eine Vielzahl von Strukturen 2 umfassen, die unter Bezugnahme auf Figur 1 vorstehend beschrieben worden sind. Dabei stoßen die Eckpunkte 24 von zwei benachbarten Strukturen 2 in Umfangsrichtung aneinander an. So sind die benachbarten Strukturen 24 auf mittlerer Höhe miteinander verbunden. Zwei benachbarte Doppelpyramidenstrukturen 2 teilen sich dementsprechend gleiche Eckpunkte 24. In Höhenrichtung, d. h. quer zur Umfangsrichtung, in welcher auch der Ring 28 umläuft, sind die jeweiligen Reihen 32 nicht miteinander verbunden. Es können zwischen 10 und 15 Reihen 32 übereinander vorgesehen sein. Je nach Pfannengröße (ca. Ø38mm-Ø80mm) und Pfannenform (rund, oval) befinden sich auf der der Eingangsebene am nächsten kommenden Reihe 32 zwischen 40 und 120 Pyramiden/ Doppelpyramiden. Die Anzahl nimmt mit zunehmender Annäherung an den Boden 34 der Pfanne 8 ab und kann bis auf Anzahl 0 zum Boden 34 auslaufen.

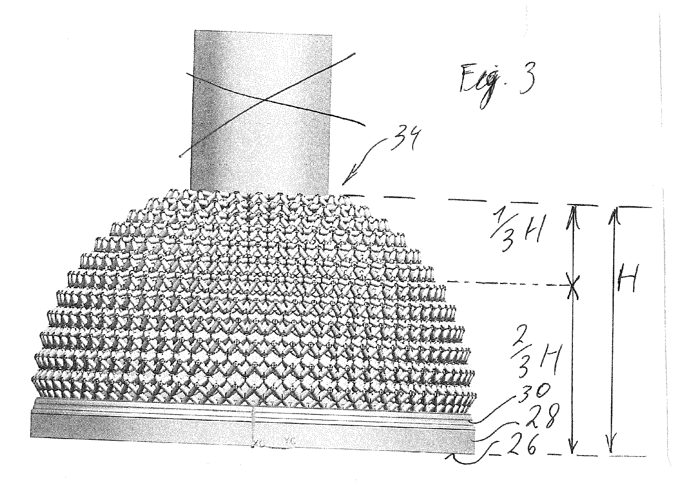

Dementsprechend bilden die Grundflächen von in Umfangsrichtung umlaufend hintereinander angeordneten Pyramiden umlaufende Gitternetzpunkte in Form der jeweiligen Eckpunkte 24 aus. Diese Gitternetzpunkte 24 sind mit einem maximalen Abstand von zwischen 1 und 2 mm zu der durchgehenden Oberfläche 6 vorgesehen. Bei dem gezeigten Ausführungsbeispiel nimmt der Abstand mit zunehmender Annäherung der umlaufenden Gitternetzpunkte 24 an einen Boden 34 der Pfanne 8 ab. Dabei ist vorliegend der Abstand bis etwa zwei Drittel der Höhe H der Pfanne 8 vorliegend identisch. Der entsprechende Abstand ist in Figur 3 mit "2/3 H" eingezeichnet. Bis zu dieser Höhe ausgehend von der Eingangsebene 26 sind die Doppelpyramiden der Oberflächengestaltung 4 jeweils identisch ausgebildet. Über die verbleibende Höhe bis zu dem Boden 34 nimmt der entsprechende Abstand der Gitternetzpunkte 24 von der durchgehenden Oberfläche 6 ab. Vorliegend beträgt dieser Abstand im Bereich des Bodens lediglich wenige Zehntel mm.

Die in dem Ausführungsbeispiel gezeigte Oberflächengestaltung 4 lässt sich einfach durch generative/additive Fertigungsverfahren, wie beispielsweise das SLM (selective laser melting) bzw. EBM (electron beam melting) bzw. DMLS (direct metal laser sintering) herstellen. Dabei wird die entsprechende Oberflächengestaltung 4 üblicherweise einstückig durch Aufschmelzen bzw. Sintern einer Pulverschuttung mit der durchgehenden Oberfläche 6 und der darunterliegenden metallischen Struktur der Pfanne 8 hergestellt. Es ergibt sich somit ein inniger Verbund zwischen der Oberflächengestaltung 4 und der darunterliegenden Basis der Pfanne 8. Durch die Doppelpyramide 20, 22 wird innerhalb derselben ein Hohlraum mit Hinterschnitten geschaffen, in welchen die Knochenzellen einwachsen und sich mit der Pfanne 8 verankern können. Die durchgehende Oberfläche 6 hat zusätzlich eine gewisse Rauigkeit, so dass dort eine vergrößerte Oberfläche und mikroskopische Vorsprünge geschaffen werden, die einen verbesserten Verbund zwischen den organischen Zellen und der Pfanne 8 erlauben.

Die Erfindung ist nicht auf das gezeigte Ausführungsbeispiel beschränkt. So kann lediglich eine einzige Pyramide vorgesehen sein, die mit ihrer unteren Spitze 16 mit der durchgehenden Oberfläche 6 verbunden und auf Höhe der Eckpunkte 24 ein drittes Quadrat aufweist, welches die Grundfläche der Pyramide bildet und die Eckpunkte verbindende, geradlinig verlaufende Stege umfasst. Reihen dieser Pyramiden können über gemeinsame Stege, die sich in Höhenrichtung der Pfanne 8 erstrecken, miteinander verbunden sein. Unterhalb dieser gemeinsamen Stege ergibt sich ebenfalls eine Zelle, in welcher Zellwachstum stattfinden kann, wobei die resultierende Zellstruktur von den die Mantelfläche der Pyramide begrenzenden Stegen überragt wird, wodurch ein Hinterschnitt gebildet ist.

Bei einer weiteren Abwandlung befindet sich die Grundfläche der Pyramide auf Höhe der durchgehenden Oberfläche 6. Die Pyramide hat eine obere Spitze 18, die den höchsten Punkt der Oberflächengestaltung bildet. Solche Pyramiden können so vorgesehen sein, dass die Grundflächen benachbarter Pyramiden unmittelbar aneinander angrenzen. Die benachbarte Pyramide kann aber auch eine Pyramidenfläche haben, die zur Hälfte in der Grundfläche der anderen Pyramide vorgesehen ist, so dass sich die Stege der beiden Pyramiden zumindest teilweise schneiden. Dadurch wird innerhalb der durch jede einzelne Pyramide gebildeten Zelle eine Unterstruktur geschaffen, durch welche die einwachsenden Knochenzellen überdeckt und damit gegenüber der Pfanne in verbesserter Weise gesichert werden. Denkbar ist auch eine Ausgestaltung, bei welcher zwei Pyramidenfelder auf der durchgehenden Oberfläche einander überlagert vorgesehen sind. Als Pyramidenfeld sei dabei eine Anordnung von Pyramiden verstanden, welche mit ihren jeweiligen Grundflächen unmittelbar nebeneinander bzw. hintereinander vorgesehen sind. Es ergibt sich ein Raster aus Grundflächen. Dieses Raster ist quadratisch oder rechteckig. In einem Schnittpunkt dieses Rasters ragen dementsprechend Stege von vier benachbarten Pyramiden von der durchgehenden Oberfläche ab. Ein zweites Pyramidenfeld, welches beispielsweise identisch zu dem ersten Pyramidenfeld ausgebildet, jedoch mit Versatz diesem überlagert ist, kann zu einer Ausgestaltung führen, bei welcher sich Stege der Pyramiden des ersten Pyramidenfeldes mit Stegen des zweiten Pyramidenfeldes schneiden. Denkbar ist beispielsweise eine Anordnung, bei welcher die Pyramiden eine quadratische Grundfläche haben und mit identischer Höhe vorgesehen sind. Die Schnittpunkte der Grundflächen von zwei Pyramiden des zweiten Pyramidenfeldes liegen dabei vorzugsweise exakt mittig in einer Grundfläche einer Pyramide des anderen Pyramidenfeldes. Dabei schneiden sich die Stege der beiden Pyramidenfelder vorzugsweise auf mittlerer Höhe der Pyramiden.

Weitere Abwandlungen sind denkbar, wie beispielsweise die Anordnung von Pyramidenfeldern geringerer Höhe in Pyramidenfeldern mit Pyramiden größerer Höhe. Allerdings sind relativ großzellige Ausgestaltungen leichter mit generativen/additiven Fertigungsverfahren herstellbar, so beispielsweise die in Figur 1 gezeigte Struktur 2 oder aber eine entsprechende Struktur mit nur einer Ebene von Pyramiden, die sich von der durchgehenden Oberfläche 6 erheben.

### Bezugszeichenliste

- 2: Struktur
- 4: Oberflächengestaltung
- 6: durchgehende Oberfläche
- 8: Pfanne
- 10: erstes Quadrat
- 12: zweites Quadrat
- 14: Steg
- 16: untere Spitze
- 18: obere Spitze
- 20: unter Pyramide
- 22: obere Pyramide
- 24: Eckpunkt
- 25: drittes Quadrat
- 26: Eingangsebene
- 28: Ring
- 30: Profilierung
- 32: Reihe
- 34: Boden

## Patentansprüche

1. Pfanne (8) einer Hüftgelenkprothese mit einer inneren Aufnahme für die drehbare Lagerung eines Oberschenkels und mit einer das Einwachsen von Knochenzellen begünstigenden Oberflächengestaltung (4), die über einer äußeren durchgehenden Oberfläche (6) der Pfanne (8) vorgesehen ist, **dadurch gekennzeichnet, dass** die Oberflächengestaltung Stege (14) umfasst, die die Mantelfläche von Pyramiden begrenzen, deren Grundfläche (24) im Wesentlichen parallel zu der durchgehenden Oberfläche (6) verläuft, von der zumindest einige Stege abragen.

2. Pfanne nach Anspruch 1, **dadurch gekennzeichnet, dass** die Stege (14) eine Doppelpyramide aufspannen, die mit ihrer Spitze mit der durchgehenden Oberfläche (6) verbunden ist.

3. Pfanne nach Anspruch 1 oder 2, **dadurch gekennzeichnet, dass** die Stege (14) geradlinig verlaufen.

4. Pfanne nach einem der Ansprüche 2 oder 3, **dadurch gekennzeichnet, dass** die untere Spitze (16) von zwei senkrecht zueinander stehenden Rechtecken (10; 12) gebildet, dass ein drittes, zu diesen ebenfalls senkrecht stehendes Rechteck (25) im Wesentlichen parallel und mit Abstand zu der durchgehenden Oberfläche (6) vorgesehen ist und dass benachbarte Doppelpyramiden (20; 22) auf Höhe des dritten Rechtecks (25) miteinander verbunden sind.

5. Pfanne nach Anspruch 4, **dadurch gekennzeichnet, dass** das dritte Rechteck (25) lediglich durch Eckpunkte (24) der beiden anderen Rechtecke gebildet ist und dass diese Eckpunkte auf Höhe des dritten Rechtecks (25) nicht durch Stege miteinander verbunden sind.

6. Pfanne nach einem der vorherigen Ansprüche, **dadurch gekennzeichnet, dass** rechteckige Grundflächen (25) von in Umfangsrichtung der Pfanne (8) umlaufend angeordneten Pyramiden miteinander verbunden sind.

7. Pfanne nach Anspruch 5 oder 6, **dadurch gekennzeichnet, dass** zwei benachbarte rechteckige Grundflächen (25) von in Umfangsrichtung nebeneinander vorgesehenen Pyramiden gemeinsame Eckpunkte (24) haben.

8. Pfanne nach einem der vorherigen Ansprüche, **dadurch gekennzeichnet, dass** die rechteckigen Grundflächen (25) von in Umfangsrichtung nebeneinander angeordneten Pyramiden umlaufende Gitternetzpunkte (24) ausbilden, die mit einem maximalen Abstand von zwischen 1 und 2 mm zu der durchgehenden Oberfläche (6) vorgesehen sind.

9. Pfanne nach Anspruch 8, **dadurch gekennzeichnet, dass** der Abstand mit zunehmender Annäherung der umlaufenden Gitternetzpunkte (24) an einen Boden (34) der Pfanne (8) abnimmt.

10. Pfanne nach Anspruch 9, **dadurch gekennzeichnet, dass** der Abstand von einer Eingangsebene (26) der Pfanne (8) bis zu einer Höhe von zwischen 50 Prozent und 70 Prozent der Gesamthöhe (H) der Pfanne (8) im Wesentlichen unverändert bleibt und darüber hinaus bis zu dem Boden kontinuierlich abnimmt.

11. Pfanne nach einem Ansprüche 7 bis 10, **dadurch gekennzeichnet, dass** für sämtliche umlaufende Gitternetzpunkte (24) das Verhältnis von Abstand zu der durchgehenden Oberfläche (6) zu Abstand benachbarter Gitternetzpunkte (24) in Umfangsrichtung und/ oder Höhenrichtung der rechteckigen Grundflächen (25) im Wesentlichen konstant ist.

12. Pfanne nach einem der Ansprüche 7 bis 10, **dadurch gekennzeichnet, dass** in Höhenrichtung benachbarte Pyramiden (20; 22) nicht miteinander verbunden sind.

13. Pfanne nach einem der vorigen Ansprüche, **dadurch gekennzeichnet, dass** die durchgehende Oberfläche (6) mit einer Rauigkeit Rₐ von zwischen 4 und 40 versehen ist.

14. Pfanne nach einem der vorigen Ansprüche, **dadurch gekennzeichnet, dass** zumindest die durchgehende Oberfläche (6) und die Stege (14) der Pyramide (20; 22) mit einem generativen/additiven Fertigungsverfahren hergestellt sind.
